# EUROPEAN PATENT SPECIFICATION

(11) **EP 1 908 435 B1**
(45) Date of publication and mention of the grant of the patent: **23.03.2011**
(21) Application number: 07019538.3
(22) Date of filing: 05.10.2007
(51) Int. Cl.: A61C 17/22, A46B 13/02, A46B 15/00, A61N 1/26, A61N 1/30

(54) **Mouth cleaning device**
Mundreinigungsvorrichtung
Dispositif de nettoyage de bouche

(30) Priority: 06.10.2006 JP 2006275682
(43) Date of publication of application: 09.04.2008
(73) Proprietor: Panasonic Electric Works Co., Ltd., Kadoma-shi Osaka (JP)
(72) Inventor: Taniguchi, Shinichi, Kadoma-shi Osaka (JP); Kishimoto, Suehisa, Kadoma-shi Osaka (JP); Kunita, Tomohiro, Kadoma-shi Osaka (JP); Shimizu, Hiroaki, Kadoma-shi Osaka (JP)
(74) Representative: Samson & Partner

(56) References cited:
- EP-A- 0 143 748
- EP-A- 1 025 776
- US-A- 4 665 921
- US-A- 5 133 102
- US-A- 5 372 501

## Description

The present invention relates to a mouth cleaning device for improving oral hygiene by flowing a minute current in a mouth.

As for a conventional mouth cleaning device, an electric toothbrush or an ion toothbrush has been known. The conventional mouth cleaning device removes plaque from teeth by flowing a minute current in a mouth, thereby enhancing cleaning effect of brushing, metabolism of oral tissues or blood flow. For example, Japanese Patent No. 2560162 describes therein a toothbrush including a head portion where bristles are arranged and a handle portion where a battery is accommodated, wherein an electrode connected with one pole of the battery is arranged on a part of a surface of the handle portion, whereas an electrode connected with the other pole of the battery is arranged on the head portion. When a user inserts the head portion into a mouth while holding the handle portion, a minute current flow through a body from a hand holding the handle portion to contact portions between the head portion and teeth or gums inside the mouth. Further, Utility Model Laid-open Application Nr. S62-30849 describes therein a toothbrush that supplies a low frequency square wave into a mouth.

However, the conventional toothbrush is not efficient in massaging gum tissues.

US 5,372,501 refers to a toothbrush for applying an electrical stimulus to the gingival region which has an electrode at the bristles and an electrode on the handle. Electronic circuitry for applying a voltage is provided. Preferably the frequency of a bipolar signal is between about 30 to 70.Hz, and more particularly between about 40 and 60 Hz. The body may be more conducive to a higher frequency signal, and so a higher frequency may also be preferred, between about 2000 and 4000 Hz, and preferably about 2800 Hz. A frequency of about 60 KHz may also be used.

US 5,133,102 discloses an electronic toothbrush that has a handle accomodating a light-emitting diode and a sound-producing device as well as an electric circuit for actuating the diode and device. A current is caused to flow through the toothbrush by grasping the brush and bringing it into contact with the teeth.

US 4,665,921 refers to a high potential generating toothbrush with a positive electrode at a handle and a negative electrode at a head with implanted bristles. There is further provided a free running type oscillator which oscillates at 2-3 kHz and produces a voltage of a maximum of about 400 volts.

EP 0 143 748 refers to a device for prophylaxis and care of tumors, caries and other diseases with notable reduction of pain, by electric and/or magnetic pulses. Electrical stimuli with a waveform similar or deriving from that of the physiological signals transmitted by nerve fibers, it is possible to reduce even to elimination of the microbial flora present. These electrical stimuli applied in the oral cavity, reduce even to elimination of germs in the deeper stratum of the dental caries. A device is disclosed, which is applied for the prophylaxis and care of dental caries and disinfecting the oral cavity. It produces a variable electrical frequency between 0,1 and 2500 Hz.

In view of the above, the present invention provides a mouth cleaning device that is efficient in massaging gum tissues.

In accordance with an embodiment of the present invention, there is provided a mouth cleaning device including: a head portion provided with bristles and an electrode and a handle portion provided with an electrode, characterized in that the mouth cleaning device further includes an electric circuit for flowing a current from one of the electrodes to the other electrode via a human body by application of a voltage to the electrodes, wherein the current is a pulse current having a frequency of about 9090 Hz to about 15000 Hz.

Preferably, the pulse current has a frequency of about 9090 Hz.

The present invention in which the pulse current having the frequency of about 9090 Hz to about 15000 Hz is made to flow is efficient in stimulating gum tissues and improving gum health.

The objects and features of the present invention will become apparent from the following description of embodiments, given in conjunction with the accompanying drawings, in which:
Fig. 1 shows a block circuit diagram of an exemplary embodiment of the present invention;
Fig. 2 is a cross sectional view of an exemplary toothbrush of the present invention;
Fig. 3 illustrates an explanatory diagram for explaining exemplary variations in hemoglobin oxygen saturation of the embodiment of the present invention to; and
Fig. 4 provides an explanatory diagram for explaining variations in an exemplary BOP (Bleeding On Probing) improvement rate of the embodiment of the present invention.

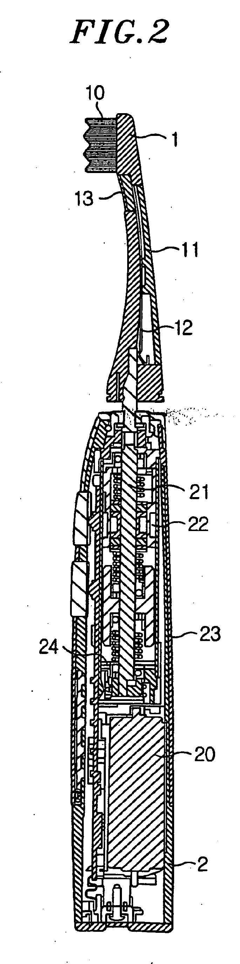

The embodiments of the present invention will be described with reference to the accompanying drawings which form a part hereof. Referring to Fig. 2, there are illustrated a head portion 1 formed by arranging bristles 10 on one end side of a shaft 11 and a handle portion 2 accommodating therein a power source (e.g., battery or the like) 20. The handle portion 2 includes therein a driving shaft 21 having one end connected with the head portion 1, an actuator 22 for moving the driving shaft 21 in an axial reciprocating motion or the like, and a circuit board 24. Moreover, the handle portion 2 has an electrode 23 on an outer surface thereof, and the head portion 1 has an electrode 13 near roots of the bristles 10.

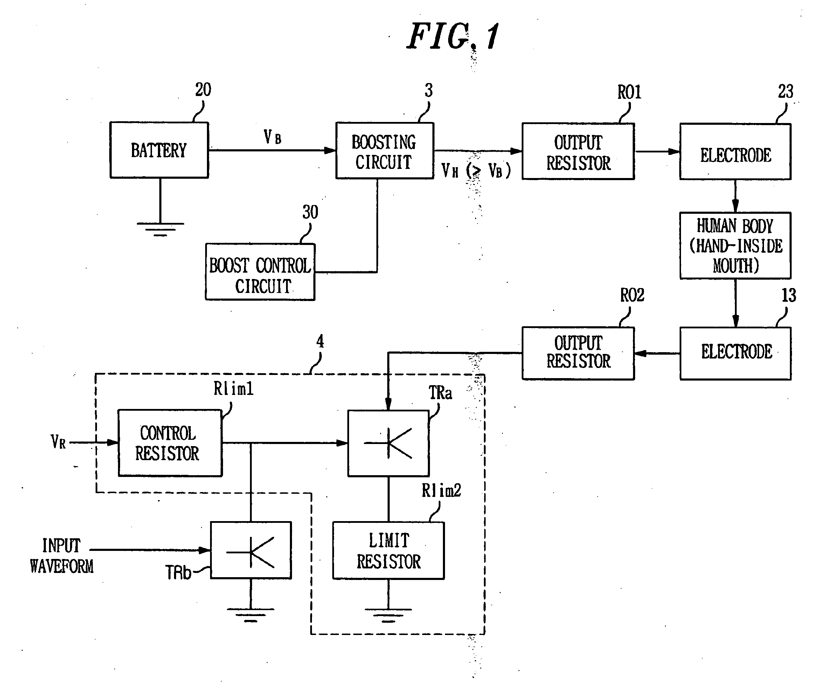

As illustrated in Fig. 1, the electrode 23 is connected with the positive pole of the battery 20 via an output resistor Ro1 and a boosting circuit 3 mounted on the circuit board 24. Further, the electrode 13 of the head portion 1 is connected with the grounded negative pole of the battery 20 via a conduction plate 12 installed inside the shaft 11, the driving shaft 21, and the circuit board 24 (i.e., an output resistor RO2, a current limit transistor TRa and limit resistor Rlim 2 mounted thereon).

The circuit board 24 has a current limit circuit 4 shown in Fig. 1, in addition to the boosting circuit 3. The current limit circuit 4, which is formed of the control resistor Rlim1, the current limit transistor TRa and a limit resistor Rlim2, generates a constant base current from a reference DC voltage V_{R} (current limit control DC voltage) via the control resistor Rlim1, and limits a collector current that can flow through the current limit transistor TRa. The control resistor Rlim1 serves to adjust the reference DC voltage V_{R}, and can variably adjust a current limit when it is configured as a variable resistor, according to individual differences in a resistance of a human body, individual differences in reaction to a current and the like. It is preferable that the maximum value of the limited current which can flow through the human body is lower than or equal to about 300 µA.

The boosting circuit 3 boosts a battery voltage V_{B} and generates a voltage Vₕ (Vₕ > V_{B}) under the control of a boost control circuit 30. This voltage Vh is preferably a voltage which allows the supply of a current of a magnitude that enables required effects to be obtained even if a resistance of a conduction path including a human body is not uniform. For example, when a current of about 100 µA needs to flow on the assumption that a maximum resistance (including a contact resistance or the like) of the path including the human body is about 150 kΩ, the voltage Vh is about 15 V (= 150 kΩ × 100 µA).

If the handle portion 2 is held and the head portion 1 is inserted into the mouth in a state where the voltage Vh generated by the boosting circuit 3 is applied to the electrodes 13 and 23, the current flows from an output resistor Ro1 to the current limit transistor TRa via the electrode 23, the human body, the electrode 13, the conduction plate 12, the driving shaft 21 and an output resistor Ro2. However, the current is limited to, e.g., about 100 µA, by the aforementioned base current. Therefore, even when the resistance of the human body is low, the current greater than or equal to about 100 µA does not flow and, also, an surge current can be suppressed.

As depicted in Fig. 1, there is provided a transistor TRb which temporarily blocking a current by reducing a base potential of the pulsed current limit transistor TRa to zero at regular intervals so that the current can flow through the human body.

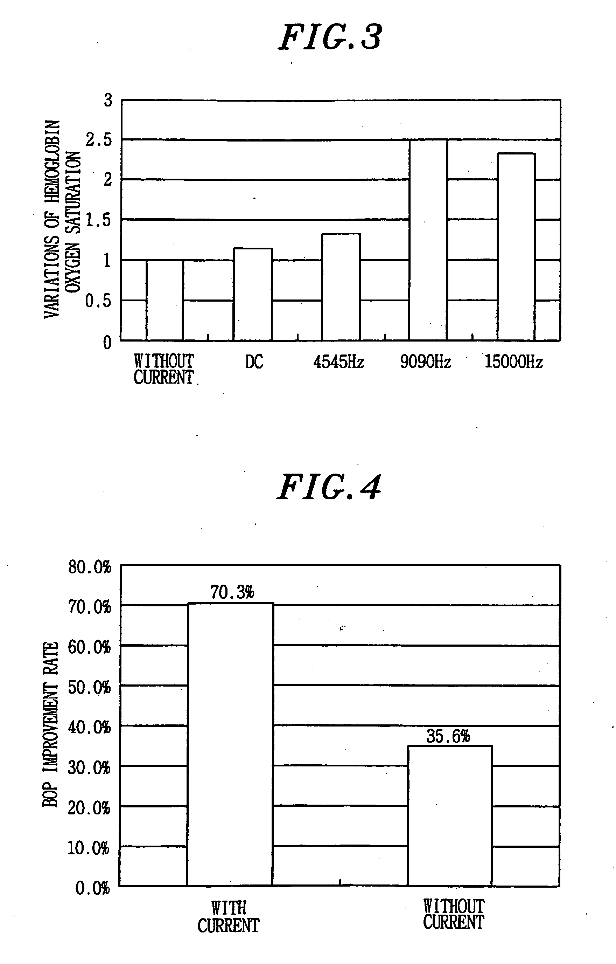

Fig. 3 shows variations in hemoglobin oxygen saturation obtained when a pulsed current, a DC current, and no current are made to flow, wherein the saturation values are normalized with respect to the value obtained when no current is made to flow. The hemoglobin oxygen saturation indicates a combination ratio between oxygen and hemoglobin in red blood cells flowing through gum blood vessels. Since the oxygen sufficiency in tissues can be monitored by measuring the hemoglobin oxygen saturation, it serves as an indicator representing a state of inflammation of gums. When the state of gum health is poor due to the inflammation of gums or the like, oxygen supply cannot meet oxygen demand and, accordingly, oxygen becomes insufficient (low hemoglobin oxygen saturation).

As clearly can be seen from Fig. 3, the saturation is higher when the DC current is made to flow than when no current is made to flow. The saturation becomes further higher when a pulsed current of about 4545 Hz is made to flow, and highest when a pulse current of 9090 Hz is made to flow. However, the saturation is smaller when a pulsed current of 15000 Hz is made to flow than when the pulsed current of 9090 Hz is made to flow.

Fig. 4 illustrates variations in BOP (Bleeding On Probing) when the pulsed current of 4545 Hz is made to flow for one month during tooth brushing and when no current is made to flow. As a result, it was found that the BOP was significantly improved when the pulsed current was made to flow. Therefore, it is clear that the gum care is effective when the pulse current of a frequency greater than or equal to about 4000 Hz is made to flow.

The electrode 13 is a negative electrode to which a single pole pulsed current is supplied to prevent elution of an electrode metal. When an electrode inserted into a mouth serves as an anode, teeth or gums serve as a cathode. In that case, a metal of the anode is eluted, and the eluted metal is deposited on the teeth or the gums serving as the anode. However, the anode can be made of a material other than a metal or a material that is not eluted. If such a material is employed as the electrode 13, the electrode 13 can be positively biased or AC-biased.

Although the head portion 1 in the above embodiment moves by the actuator 22, the actuator 22 (or another driving unit) can be omitted.

While the invention has been shown and described with respect to the embodiments, it will be understood by those skilled in the art that various changes and modification may be made without departing from the scope of the invention as defined in the following claims.

## Claims

1. A mouth cleaning device comprising:
a head portion (1) provided with bristles (10) and an electrode (13); and
a handle portion (2) provided with an electrode (23), **characterized in that** the mouth cleaning device further comprises;
an electric circuit (24) for flowing a current from one of the electrodes (13 or 23) to the other electrode (23 or 13) via a human body by application of a voltage to the electrodes (13, 23),
wherein the current is a pulse current having a frequency of about 9090 Hz to about 15000 Hz.

2. The mouth cleaning device of claim 1, wherein the pulse current has a frequency of about 9090 Hz.

## Patentansprüche

1. Mundreinigungsvorrichtung, umfassend:
einen Kopfabschnitt (1), der mit Bürsten (10) und einer Elektrode (13) versehen ist; und
einen Griffabschnitt (2), der mit einer Elektrode (23) versehen ist, **dadurch gekennzeichnet, dass** die Vorrichtung ferner umfasst:
eine elektrische Schaltung (24), um einen Strom von einer der Elektroden (13 oder 23) zur anderen Elektrode (23 oder 13) über einen menschlichen Körper durch Anlegen einer Spannung an den Elektroden (13, 23) fließen zu lassen,
wobei der Strom ein Pulsstrom ist, der eine Frequenz von ungefähr 9090 Hz bis ungefähr 15000 Hz aufweist.

2. Mundreinigungsvorrichtung gemäß Anspruch 1, wobei der Pulsstrom eine Frequenz von ungefähr 9090 Hz aufweist.

## Revendications

1. Dispositif de nettoyage buccal comprenant :
une partie de tête (1) dotée de poils (10) et d'une électrode (13) ; et
une partie de manche (2) dotée d'une électrode (23), **caractérisé en ce que** le dispositif de nettoyage buccal comprend en outré :
un circuit électrique (24) pour faire circuler un courant allant de l'une des électrodes (13 ou 23) jusqu'à l'autre électrode (23 ou 13), via un corps humain par l'application d'une tension sur les électrodes (13 , 23),
dans lequel le courant est un courant à impulsions ayant une fréquence d'environ 9 090 Hz à environ 15 000 Hz.

2. Dispositif de nettoyage buccal selon la revendication 1, dans lequel le courant à impulsions a une fréquence d'environ 9 090 Hz.
